# EUROPEAN PATENT SPECIFICATION

(11) **EP 2 902 051 B1**
(45) Date of publication and mention of the grant of the patent: **24.10.2018**
(21) Application number: 12885885.9
(22) Date of filing: 26.09.2012
(51) Int. Cl.: A61M 5/145, A61M 5/00, A61M 5/168

(54) **SYRINGE PUMP**
SPRITZENPUMPE
POMPE DE SERINGUE

(43) Date of publication of application: 05.08.2015
(73) Proprietor: Terumo Kabushiki Kaisha, Tokyo 151-0072 (JP)
(72) Inventor: UEMURA, Tomoko, Ashigarakami-gun Kanagawa 259-0151 (JP)
(74) Representative: Casalonga
(86) International application number: PCT/JP2012/006165
(87) International publication number: WO 2014/049647

(56) References cited:
- EP-A2- 1 110 569
- EP-A2- 1 240 913
- WO-A2-01/72357
- JP-A- S6 468 274
- JP-A- H08 191 591
- JP-A- 2001 245 978
- JP-A- 2001 245 978
- JP-A- 2002 113 099
- JP-A- 2002 272 839
- US-A- 4 137 913
- US-A1- 2003 205 587
- US-A1- 2010 217 193
- US-B1- 6 362 591

## Description

### Technical Field

The present invention relates to a syringe pump in which a syringe is mounted and a drug solution inside the syringe is delivered to a patient.

### Background Art

A syringe pump is used in an intensive care unit (ICU) or the like, for example, and is adopted to perform delivering treatment of a drug solution such as anticancer drugs, anesthetics, chemotherapeutics, blood preparations or the like, and nutrients or the like to a patient with high accuracy for a relatively long period of time. The syringe pump is accurate and excellent in a flow control of the drug solution compared to other infusion pumps.

In a care unit or an operating room using the syringe pump, multiple types of syringes respectively having different capacities are prepared in advance. A health care worker selects a syringe of a necessary capacity from the multiple types of syringes, and the syringe of the selected capacity is mounted in the above-described syringe pump. As the syringe is mounted in a containing portion of the syringe pump, a main body flange of a syringe main body is held, and a plunger flange of a syringe plunger is grasped by a slider serving as a movement member. Accordingly, as a motor of the syringe pump is driven, the slider gradually presses the syringe plunger toward the syringe main body, and then, the syringe plunger pushes out a drug solution inside the syringe main body. Thus, it is possible to deliver the drug solution to a patient through a tube (refer to PTL 1).

### Citation List

### Patent Literature

PTL 1: JP-A-2010-88564

Pertinent syringe pumps are described in US 2003/205587 A1 and US 2010/217193 A1.

### Summary of Invention

### Technical Problem

Incidentally, as a health care worker turns on a power switch of a syringe pump and causes a motor of the syringe pump to operate, a slider gradually presses a syringe plunger toward a syringe main body so as to push out a drug solution inside the syringe main body. Thus, it is possible to deliver the drug solution to a patient through a tube. However, depending on a positional relationship between the syringe pump and a patient, a portion of the tube may be bent or twisted, thereby resulting in a state where the tube is occluded. If the tube is in an occlusion state, a drug stops flowing, and thus, the drug cannot be safely delivered to the patient.

In this case, if the health care worker straightens bending or twisting of the tube, since the occlusion state of the tube is instantly relieved, there is a concern that the drug may abruptly flow inside the tube in which the occlusion state has been released, thereby being delivered to the patient.

Therefore, the present invention aims to provide a syringe pump which can discontinue delivery of a drug while preventing the drug from abruptly flowing inside a tube if an occlusion state of the tube is released by straightening bending or twisting of the tube, and thus, the safety of an operation of delivering a drug can be enhanced.

The invention is defined by the subject-matter of independent claim 1.

### Solution to Problem

According to the present invention, a syringe pump delivers a drug inside a syringe to a patient via a tube by pressing a syringe plunger of the syringe filled with the drug. The syringe pump includes a syringe placement portion in which a syringe main body of the syringe is placed (set); a drive motor; a movement member that presses the syringe plunger in accordance with a clockwise rotation operation of the drive motor so as to deliver the drug inside the syringe to the patient; a pressure sensor that detects pressure while pressing the syringe plunger, in order to detect that the tube is in an occluded state; and a control unit that performs occlusion mitigation treatment by starting counterclockwise rotation of the drive motor when pressure detected by the pressure sensor exceeds a predetermined occlusion judgement standard value, and subsequently stops a counterclockwise rotation operation of the drive motor if any one of a plurality of predetermined conditions for stopping the counterclockwise rotation is fulfilled. The plurality of predetermined conditions for stopping the counterclockwise rotation are the following (1) to (4):
(1) Occlusion pressure is equal to or less than a predetermined value (50 mmHg),
(2) "Quantity of counterclockwise rotations" determined for each syringe reaches a predetermined number of steps,
(3) Integrated quantity is equal to or less than integrated quantity at the time of starting delivering a drug, and
(4) Integrated quantity is zero.

According to the configuration, the pressure sensor detects pressure at the time of pressing the syringe plunger, in order to detect that the tube is in an occluded state. The control unit causes the drive motor to perform a counterclockwise rotation operation when pressure detected by the pressure sensor exceeds the predetermined occlusion judgment standard value, and the control unit causes the drive motor to stop the counterclockwise rotation operation when pressure detected by the pressure sensor becomes equal to or less than the predetermined occlusion judgement standard value. Accordingly, delivery of a drug can be discontinued while preventing the drug from abruptly flowing inside the tube if an occlusion state of the tube is released by straightening bending or twisting of the tube. Thus, it is possible to enhance the safety of an operation of delivering a drug.

It is preferable that the drive motor is a step motor, and the pressure sensor is placed in the movement member.

According to the configuration, the step motor is adopted as the drive motor. Therefore, the drive motor can be easily shifted from a clockwise rotation operation to a counterclockwise rotation operation. Moreover, the pressure sensor can directly receive pressure from the syringe plunger. Thus, it is possible to easily detect the occlusion state of the tube.

It is preferable that the control unit causes excitation of the counterclockwise rotation operation of the drive motor to be maintained for a certain period of time when stopping the counterclockwise rotation operation of the drive motor.

According to the configuration, a rotor can be prevented from rotating any further counterclockwise due to inertia of the rotor when stopping the counterclockwise rotation operation of the rotor of the drive motor. Thus, a drug inside the tube is unlikely to be subjected to unnecessary aspirating.

It is preferable that the syringe pump includes a notification unit that issues notification regarding stopping of the drive motor if the drive motor stops.

According to the configuration, the notification unit issues notification regarding stopping of the drive motor. Thus, it is possible for a health care worker to confirm discontinuance of the operation of delivering a drug caused by the stopping of the drive motor.

It is preferable that the notification unit is a speaker which performs an acoustic announcement regarding stopping of the drive motor.

According to the configuration, the speaker issues acoustic notification regarding stopping of the drive motor. Thus, it is possible for a health care worker to acoustically confirm discontinuance of the operation of delivering a drug caused by the stopping of the drive motor.

It is preferable that the notification unit is a display unit which performs a display regarding stopping of the drive motor.

According to the configuration, the display unit issues notification regarding stopping of the drive motor. Thus, it is possible for a health care worker to visually confirm discontinuance of the operation of delivering a drug caused by the stopping of the drive motor.

It is preferable that the display unit displaying information and an operation panel section having an operation button are placed at an upper portion of a main body of the syringe pump, and a syringe setting portion and the movement member are placed at a lower portion of the main body of the syringe pump.

According to the configuration, a health care worker can perform the task of delivering a drug solution from the syringe while confirming information on the display unit at the upper portion of the main body. Thus, a health care worker can operate the operation button of the operation panel section while confirming the information on the display unit at the upper portion of the main body.

### Advantageous Effects of Invention

According to the present invention, it is possible to provide a syringe pump which can discontinue delivery of a drug while preventing the drug from abruptly flowing inside a tube if an occlusion state of the tube is released by straightening bending or twisting of the tube, and thus, the safety of an operation of delivering a drug can be enhanced.

### Brief Description of Drawings

[Fig. 1] Fig. 1 is a perspective view of a preferable embodiment of a syringe pump of the present invention.
[Fig. 2] Fig. 2 is a perspective view when the syringe pump illustrated in Fig. 1 is seen in a W-direction.
[Fig. 3] Fig. 3 is a perspective view illustrating examples of syringes in multiple sizes.
[Fig. 4] Fig. 4 is a diagram illustrating an example of an electrical configuration example of the syringe pump.
[Fig. 5] Fig. 5 is a perspective view partially illustrating a syringe setting portion and a syringe plunger drive unit shown in Fig. 2.
[Fig. 6] Fig. 6 is an enlarged perspective view when the syringe setting portion and the syringe plunger drive unit partially illustrated in Fig. 5 are seen in an E-direction.
[Fig. 7] Fig. 7 is an exploded perspective view illustrating the syringe plunger drive unit, a boot, or the like.
[Fig. 8] Fig. 8 is a perspective view illustrating the boot being shifted from a state of being stretched to the maximum to a state of being contracted.
[Fig. 9] Fig. 9 is a diagram illustrating a structural example of a drive motor.
[Fig. 10] Fig. 10 is a diagram illustrating an example of a pulse input current waveform applied to the drive motor illustrated in Fig. 9.
[Fig. 11] Fig. 11 is a flow chart illustrating an operational example of the drive motor of the syringe pump.

### Description of Embodiments

Hereinafter, a preferable embodiment of the present invention will be described in detail with reference to the drawings.

The below-described embodiment is a suitable specific example of the present invention and is applied with various limitations which are technically preferable. However, the scope of the present invention is not limited to the aspects thereof unless there is disclosure particularly limiting the present invention in the following descriptions.

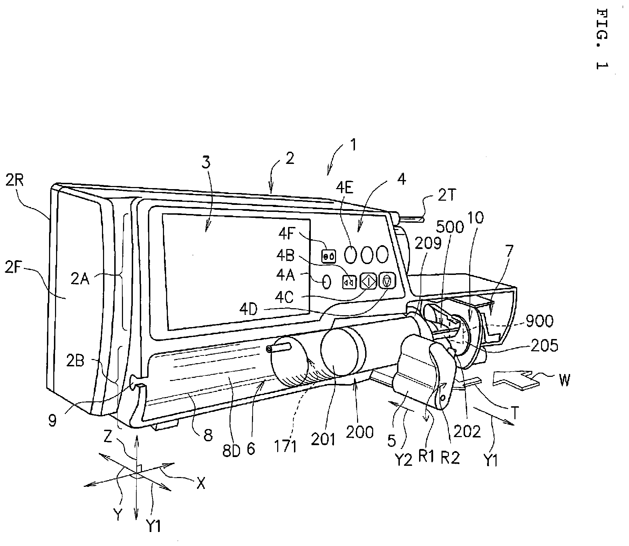

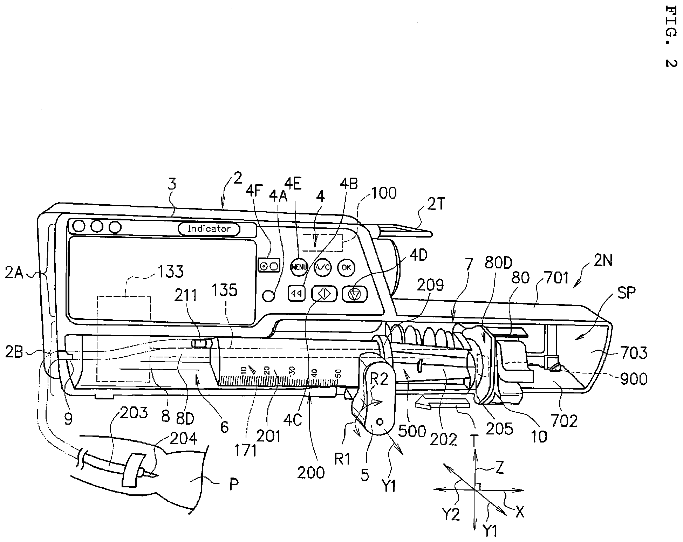

Fig. 1 is a perspective view of the embodiment of a syringe pump of the present invention. Fig. 2 is a perspective view when the syringe pump illustrated in Fig. 1 is seen in a W-direction. A syringe pump 1 illustrated in Figs. 1 and 2 is a continuous microinjection pump which is used in an intensive care unit or the like, for example, and is adopted to perform continuous microinjection treatment of a drug solution, for example, anticancer drugs, anesthetics, chemotherapeutics, blood preparations or the like, and nutrients or the like to a patient with high accuracy for a relatively long period of time.

As illustrated in Figs. 1 and 2, in the syringe pump 1, a syringe main body 201 of a syringe 200 filled with a drug solution, for example, can be fixedly mounted by using a clamp 5 so as not to move. Since a drive motor 133 of a syringe plunger drive unit 7 illustrated in Fig. 2 causes a feed screw 135 to rotate, a syringe plunger pressuring member 10 of the syringe plunger drive unit 7 can pressure a syringe plunger 202 of the syringe 200 toward the syringe main body 201 in a T-direction. As the drive motor 133, for example, a two-phase quadrupole-type step motor (a stepping motor) can be preferably used.

Accordingly, as illustrated in Fig. 2, a drug solution inside the syringe main body 201 can be properly delivered to a patient P via a tube 203 and an indwelling needle 204. The tube 203 is a line for delivering a drug 171, and is a tube, for example, made with a flexible thermoplastic resin such as soft vinyl chloride or the like. The tube 203 connects an outlet portion 211 at a distal end of the syringe main body 201 and the indwelling needle 204 to each other.

The syringe plunger pressuring member 10 is an example of a movement member for pressing and moving the syringe plunger 202 toward the syringe main body 201 while a plunger flange 205 of the syringe plunger 202 is grasped, and is also referred to as a slider.

As illustrated in Fig. 2, the syringe pump 1 has a casing 2, and the casing 2 is integrally molded with a molding resin material resistant to chemicals. As illustrated in Fig. 1, the casing 2 is assembled by joining a front cover 2F and a rear cover 2R, thereby being configured to be a case body having a drip-proof performance. Accordingly, as described below, the syringe pump 1 has a splash-proof and drip-proof treatment structure so as to be able to prevent its inside from being infiltrated even when the drug solution, moisture, or the like is splashed thereon.

Firstly, each of elements equipped in the casing 2 of the syringe pump 1 will be described.

As illustrated in Fig. 2, the syringe pump 1 has the casing 2 and a grip 2T. A display unit 3 and an operation panel section 4 are placed in an upper portion 2A of the casing 2. A syringe setting portion 6 and the syringe plunger drive unit 7 are placed in a lower portion 2B of the casing 2. Accordingly, a health care worker can perform the task of delivering the drug solution from the syringe 200 while visually confirming information contents displayed in color on the display unit 3 at the upper portion 2A of the casing 2. Then, a health care worker can operate an operation button of the operation panel section 4 while confirming the information contents displayed in color on the display unit 3 of the casing 2.

The display unit 3 illustrated in Figs. 1 and 2 is a color liquid crystal display apparatus (LCD) which can perform a color graphic display. The display unit 3 is placed at an upper left position of the upper portion 2A of the casing 2, that is, above the syringe setting portion 6 and the syringe plunger drive unit 7. The operation panel section 4 is placed on the right side of the display unit 3 at the upper portion 2A of the casing 2. In the illustrated example of the operation panel section 4, a lamp 4A configured to include an LED or the like in order to work as an operation indicator (being lit or blinking green during a normal operation, and being lit or blinking red at the time of abnormality), a fast-delivery switch button 4B, a start switch button 4C, a stop switch button 4D, a menu selection button 4E, a power switch 4F, and the like are placed as the operation buttons.

The upper portion 2A of the casing 2 illustrated in Fig. 2 is the upper half portion of the casing 2. The lower portion 2B of the casing 2 is the lower half portion of the casing 2. In the examples illustrated in Figs. 1 and 2, the syringe setting portion 6 and the syringe plunger drive unit 7 are placed side by side along an X-direction. For example, a syringe 200 of a necessary capacity is selected from the syringes having multiple types of capacities differing from one another, and the selected syringe 200 can be mounted in the syringe setting portion 6 in a detachably fit manner.

The syringe setting portion 6 illustrated in Figs. 1 and 2 has an accommodating portion 8 containing the syringe main body 201, the clamp 5, and a main body flange pressing portion 500 for grasping a main body flange 209 in a detachably fit manner. The accommodating portion 8 has a concave-type syringe main body holding unit 8D. A tube fixing portion 9 for detachably pinching the flexible tube 203 is formed on a wall portion at an end portion of the accommodating portion 8 on the left. The tube fixing portion 9 is a groove portion which pinches and fixes a portion of the tube 203 so as not to move as illustrated in Fig. 2.

In Figs. 1 and 2, when a health care worker detaches the syringe 200 from the syringe setting portion 6 by operating the clamp 5, for example, the clamp 5 is pulled against the force of a spring (not illustrated) in a Y1-direction (a front direction) and is turned by 90 degrees in an R1-direction. Then, the clamp 5 is separated from an outer circumferential surface of the syringe main body 201. Accordingly, the syringe main body 201 can be detached from the syringe main body holding unit 8D of the accommodation portion 8 by cancelling a fixed state caused by the clamp 5, and the tube 203 can be detached from the inside of the tube fixing portion 9.

When the syringe 200 is contained in the accommodation portion 8 of the syringe setting portion 6 by operating the clamp 5 so as to be attached thereto, the clamp 5 is pulled against the force of the spring (not illustrated) in the Y1-direction, and is turned by 90 degrees in an R2-direction, thereby returning in a Y2-direction due to the force of the spring. Thus, the syringe main body 201 can be contained inside the syringe main body holding unit 8D of the containing portion 8, and the tube 203 can be fixed by the clamp 5 while being fitted inside the tube fixing portion 9.

As illustrated in Figs. 1 and 2, as the syringe main body 201 is contained and mounted in the syringe main body holding unit 8D of the accommodating portion 8, the syringe plunger 202 is placed inside the syringe plunger drive unit 7. The syringe plunger drive unit 7 includes the syringe plunger pressuring member 10. As the drive motor 133 in Fig. 2 operates in response to a command from a control unit and the feed screw 135 rotates, the syringe plunger pressuring member 10 gradually presses the plunger flange 205 of the syringe plunger 202 toward the syringe main body 201 along the T-direction. The feed screw 135 is a guide member for guiding the syringe plunger pressuring member 10 by gradually pressing the syringe plunger pressuring member 10 toward the containing portion 8.

Accordingly, a drug solution inside the syringe main body 201 can be delivered to the patient P through the tube 203 and the indwelling needle 204 with high accuracy for a relatively long period of time. Note that, the X-direction, a Y-direction, and a Z-direction in Figs. 1 and 2 are orthogonal to one another. The Z-direction is a vertical direction.

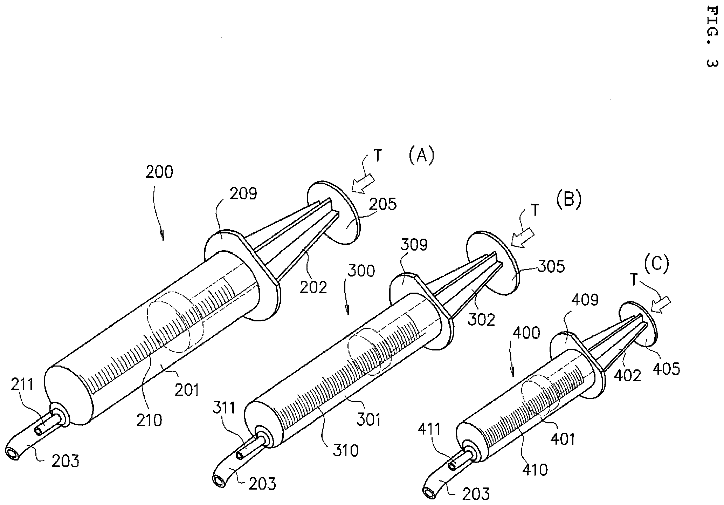

Fig. 3 is a perspective view illustrating examples of the above-described syringes in multiple sizes. Figs. 1 and 2 illustrate an example in which the syringe 200 having the largest capacity for a drug solution is fixed. As illustrated in Fig. 3(A), the syringe 200 having the largest capacity for a drug solution includes the syringe main body 201 and the syringe plunger 202. The syringe main body 201 has the main body flange 209, and the syringe plunger 202 has the plunger flange 205. A scale 210 for a drug solution is formed in the syringe main body 201. One end portion of the flexible tube 203 is detachably connected to the outlet portion 211 of the syringe main body 201.

As illustrated in Fig. 3(B), a syringe 300 having an intermediate capacity for a drug solution includes a syringe main body 301 and a syringe plunger 302. The syringe main body 301 has a main body flange 309, and the syringe plunger 302 has a plunger flange 305. A scale 310 for a drug solution is formed in the syringe main body 301. The one end portion of the flexible tube 203 is detachably connected to an outlet portion 311 of the syringe main body 301.

As illustrated in Fig. 3(C), a syringe 400 having the smallest capacity for a drug solution includes a syringe main body 401 and a syringe plunger 402. The syringe main body 401 has a main body flange 409, and the syringe plunger 402 has a plunger flange 405. A scale 410 for a drug solution is formed in the syringe main body 401. The one end portion of the flexible tube 203 is detachably connected to an outlet portion 411 of the syringe main body 401.

For example, the syringe 200 illustrated in Fig. 3(A) has a capacity of 10 mL for a drug solution. For example, the syringe 300 illustrated in Fig. 3(B) has a capacity of 5 mL for a drug solution. For example, the syringe 400 illustrated in Fig. 3(C) has a capacity of 2.0 mL for a drug solution.

As illustrated in Fig. 3, each of the syringe main bodies 201, 301, and 401 of the syringes 200, 300, and 400 is different in size from one another (volume, outer diameter, or the like) . Each of the syringe main bodies 301 and 401 of the syringes 300 and 400 can be contained in the syringe main body holding unit 8D of the containing portion 8 and so as to be fixed thereto similar to the syringe 200 illustrated in Figs. 1 and 2. Fig. 3 illustrates three types of the syringes. However, without being limited thereto, a capacity for a drug solution which the syringe can contain may range from 2.0 mL to 50 mL, for example, 20 mL, 30 mL, 50 mL, and the like. The capacity of the syringe which can be placed (set) with respect to the syringe pump 1 can be arbitrarily selected.

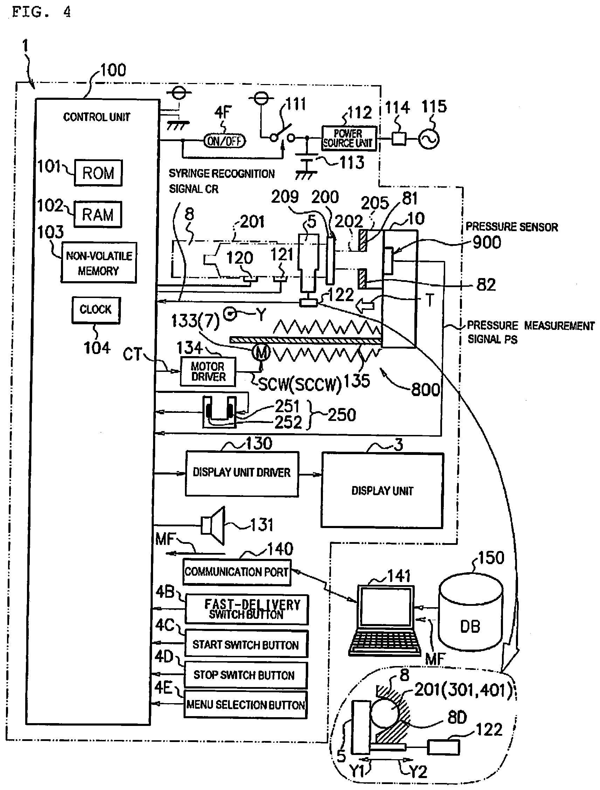

Subsequently, an example of an electrical configuration of the syringe pump 1 illustrated in Figs. 1 and 2 will be described with reference to Fig. 4.

In Fig. 4, the syringe pump 1 has a control unit (a computer) 100 which controls overall operations. The control unit 100 is a one-chip microcomputer, for example, and has a ROM (read only memory) 101, a RAM (random access memory) 102, a non-volatile memory 103, and a clock 104. The clock 104 can correct a current time, can acquire the current time, can measure an elapsed time for predetermined task of delivering a drug, and can measure a standard time for controlling a speed of delivering a drug, for example, by performing predetermined operations.

The power switch button 4F and a switch 111 are connected to the control unit 100 illustrated in Fig. 4. The switch 111 allows a power source to be supplied to the control unit 100 from either a power source converter 112 or a rechargeable battery 113, that is, a lithium-ion battery for example, by switching between the power source converter 112 and the rechargeable battery 113. The power source converter 112 is connected to a commercial AC power source 115 through a power plug 114. In Fig. 4, for example, a pair of detection switches 120 and 121 is placed inside the containing portion 8. It is preferable that the detection switches 120 and 121 detect whether or not the syringe main body 201 of the syringe 200 is properly placed inside the containing portion 8, thereby notifying the control unit 100 thereof. In other words, the detection switches 120 and 121 are detachment detection sensors for the syringe main body detecting a state when the syringe main body is not properly placed inside the containing portion 8.

A potentiometer 122 which is a clamp sensor illustrated in Fig. 4 is connected to the clamp 5. The potentiometer 122 is a syringe type discrimination sensor discriminating the sizes of the syringes 200, 300, and 400 illustrated in Fig. 3 (that is, differences in the outer diameters of outer cylinders in accordance with their volume) . The potentiometer 122 detects an amount of movement of the clamp 5 when the clamp 5 moves with respect to the Y2-direction while the syringe main body 201 is clamped by the clamp 5. Accordingly, the potentiometer 122 transmits a detection signal so as to notify the control unit 100 of the type of the capacity of the syringe main body clamped by the clamp 5 among the syringe main bodies 201, 301, and 401. The control unit 100 acquires an amount of movement of the clamp 5 with respect to the Y-direction through the detection signal from the potentiometer 122, and thus, for example, it is possible to discriminate that which syringe is mounted among the syringe main bodies 201, 301, and 401 in multiple types of volume illustrated in Fig. 3.

Note that, depending on the manufacturer thereof, even though the syringe has the same volume, each of the syringe main bodies 201, 301, and 401 is slightly different in size (volume, outer diameter, length, or the like) and the syringe plungers 202, 302, and 403.

Moreover, force that pressurizes each of the plunger flanges 205, 305, and 405 in order to send a drug is also slightly different from one another.

Therefore, data related to occlusion pressure (for each syringe) corresponding to the manufacturer and each size of the syringes is stored in a storage unit.

As the drive motor 133 of the syringe plunger drive unit 7 illustrated in Fig. 4 is driven by a motor driver 134 in response to a command from the control unit 100, the drive motor 133 causes the feed screw 135 to rotate, thereby moving the syringe plunger pressurizing member 10 in the T-direction. Accordingly, the syringe plunger pressurizing member 10 pressurizes the syringe plunger 202 in the T-direction, thereby properly delivering a drug solution inside the syringe main body 201 illustrated in Fig. 2 to the patient P through the tube 203 via the indwelling needle 204.

A two-phase step motor or a three-phase step motor is used as the drive motor 133. However, a motor drive 134 supplies an input current waveform SCW for clockwise rotation or an input current waveform SCCW for counterclockwise rotation described below to the drive motor 133 in response to a command CT from the control unit 100.

In Fig. 4, a display unit driver 130 drives the display unit 3 in response to a command from the control unit 100, thereby causing various types of information, notification contents and the like to be displayed. A speaker 131 acoustically issues notification regarding various notification contents in response to a command from the control unit 100. The control unit 100 is capable of interactive communication with respect to an external computer 141, for example, a desktop computer through a communication port 140. The external computer 141 is connected to a drug solution database (DB) 150, and drug solution information MF stored in the drug solution database 150 can be acquired by the control unit 100 via the external computer 141 so as to be stored in the non-volatile memory 103 of the control unit 100. The control unit 100 can cause the display unit 3 to display the drug solution information MF or the like based on the stored drug solution information MF.

Note that, as the drug information MF, the name of a drug manufacturer, the name of a drug, the upper and lower limits of quantity (mL) of a drug dosage, the upper and lower limits of a flow rate (mL/h), contraindication information, or the like can be exemplified.

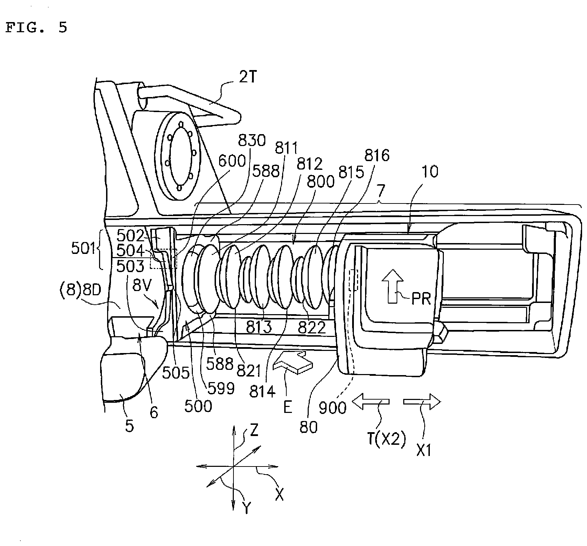

In Fig. 4, the fast-delivery switch button 4B, the start switch button 4C, the stop switch button 4D, the menu selection button 4E, and the power switch 4F are electrically connected to the control unit 100. In addition, a photo-coupler sensor 250 is electrically connected to the control unit 100 as a detector for detecting that the main body flange 209 is grasped by the main body flange pressing portion 500 (refer to Fig. 5). The photo-coupler sensor 250 has a light emitting device 251 and a light receiving device 252 which receives light from the light emitting device 251.

As illustrated in Fig. 4, the syringe plunger pressuring member 10 is provided with a pressure sensor 900. For example, the pressure sensor 900 can employ a strain sensor or the like. When pressing the syringe plunger 202 in the T-direction while the syringe plunger pressuring member 10 grasps the plunger flange 205 of the syringe plunger 202 of the syringe 200, the pressure sensor 900 can detect a change in pressure received from the plunger flange 205. Accordingly, it is possible to detect an occlusion state in which a portion of the tube 203 illustrated in Fig. 2 is bent or twisted due to a problem in a positional relationship between the syringe pump 1 illustrated in Fig. 2 and the patient P.

Note that, the pressure sensor 900 is not necessarily provided in the syringe plunger pressuring member 10.

If there is no problem in the positional relationship between the syringe pump 1 illustrated in Fig. 2 and the patient P and no portion of the tube 203 is bent or twisted, there is no occurrence of occlusion inside the tube 203. Accordingly, the syringe plunger 202 smoothly moves in the T-direction when the syringe plunger pressuring member 10 presses the syringe plunger 202 in the T-direction. As a result, the pressure sensor 900 is only lightly pressed by the plunger flange 205. Therefore, a value of a pressure measurement signal PS output from the pressure sensor 900 illustrated in Fig. 4 is equal to or less than a predetermined occlusion pressure judgement standard value LC.

In contrast, for example, depending on the positional relationship between the syringe pump 1 illustrated in Fig. 2 and the patient P, there is a case where a portion of the tube 203 may be bent or twisted, resulting in occlusion inside the tube 203. As the tube 203 is in an occlusion state, the drug 171 cannot be delivered to the patient P. Accordingly, when the syringe plunger pressurizing member 10 presses the syringe plunger 202 in the T-direction, the syringe plunger 202 is less likely to move in the T-direction. As a result, the pressure sensor 900 is strongly pressed by the plunger flange 205. Therefore, the pressure measurement signal PS output from the pressure sensor 900 illustrated in Fig. 4 exceeds the predetermined occlusion pressure judgement standard value LC.

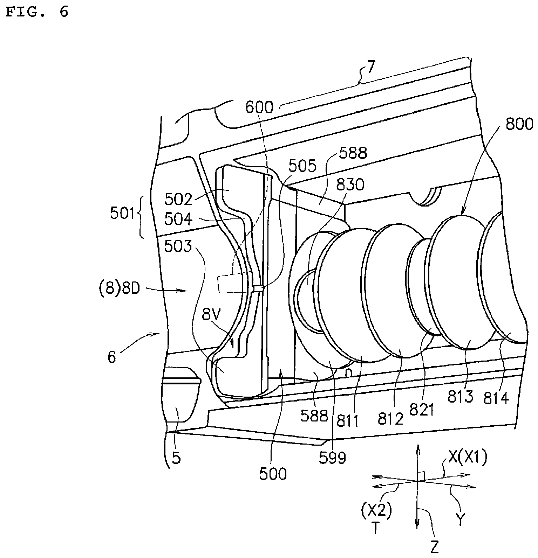

Subsequently, the detailed structure of the syringe setting portion 6 will be described with reference to Figs. 5 and 6.

Fig. 5 is a perspective view partially illustrating the syringe setting portion 6 and the syringe plunger drive unit 7 shown in Fig. 2. Fig. 6 is an enlarged perspective view when the syringe setting portion 6 and the syringe plunger drive unit 7 partially illustrated in Fig. 5 are seen in an E-direction.

The syringe setting portion 6 illustrated in Fig. 5 has the accommodating portion 8 containing the syringe main body 201, the clamp 5, the main body flange pressing portion 500 which presses and grasps the main body flange 209 (refer to Fig. 3) of the syringe 200, and a main body flange detection unit 600.

As illustrated in Figs. 1 and 2, as an example, the syringe main body 201 of the syringe 200 is set in the syringe setting portion 6, and the syringe main body 201 of the syringe 200 is fixed by using the clamp 5. As illustrated in Figs. 5 and 6, the accommodating portion 8 of the syringe setting portion 6 is a concave portion which can contain the syringe main body 201, and an axial direction of the accommodating portion 8 coincides with the X-direction. A portion of the outer circumferential surface of the syringe main body 201 is in close contact with an inner surface of the syringe main body holding unit 8D of the accommodating portion 8, and the remaining portion of the outer circumferential surface of the syringe main body 201 is exposed to the outside. The main body flange detection unit 600 includes the photo-coupler sensor 250 illustrated in Fig. 4.

The shape of the main body flange pressing portion 500 will be described with reference to Figs. 5 and 6. The shape of the main body flange pressing portion 500 is merely an example, and thus, the main body flange pressing portion 500 is not limited to the shape thereof. The main body flange pressing portion 500 is placed along a plane formed in the Y-direction and the Z-direction.

As illustrated in Figs. 5 and 6, the main body flange pressing portion 500 has a distal end portion 501 and two connection portions 588. It is preferable that the distal end portion 501 has two introduction portions 502 and 503, and a concave portion 504 which is formed between the introduction portions 502 and 503.

Accordingly, as illustrated in Figs. 1 and 2, a health care worker can easily insert the main body flange 209 of the syringe 200 into a place between an inner surface of the main body flange pressing portion 500 and a right side surface portion 8V of the syringe main body holding unit 8D along the Y2-direction illustrated in Fig. 2 by using the two introduction portions 502 and 503 illustrated in Fig. 6. Accordingly, the main body flange pressing portion 500 can reliably fix the main body flange 209. As illustrated in Figs. 5 and 6, a substantially circular hole portion 599 is formed between the connection portions 588 on both sides. As illustrated in Fig. 6, the hole portion 599 is formed in order to allow the below-described boot 800, that is, a cover member to pass through. Accordingly, when the syringe plunger 202 illustrated in Fig. 2 is pressed toward the syringe main body 201 so as to deliver a drug solution inside the syringe main body 201, the boot 800 is elastically deformed so as to be contractible without coming into contact with the main body flange pressing portion 500.

Returning to Fig. 6, in a state where the syringe pump 1 is used, the introduction portion 502 is positioned above with respect to the Z-direction, and the introduction portion 503 is positioned below. As illustrated in Fig. 6, it is preferable that a concave portion 505 which is smaller than the concave portion 504 is formed at a central position thereof. The small concave portion 505 is a groove portion in which a portion of a blade portion of the syringe plunger 202 illustrated in Fig. 2 is inserted while the syringe 200 is mounted. Accordingly, the blade portion of the syringe plunger 202 is unlikely to get on a surface of the concave portion 504 of the main body flange pressing portion 500. Therefore, the syringe 200 can be reliably fixed to a predetermined position. The same configuration can be applied not only to the syringe 200 but also to the syringes 300 and 400 illustrated in Fig. 3.

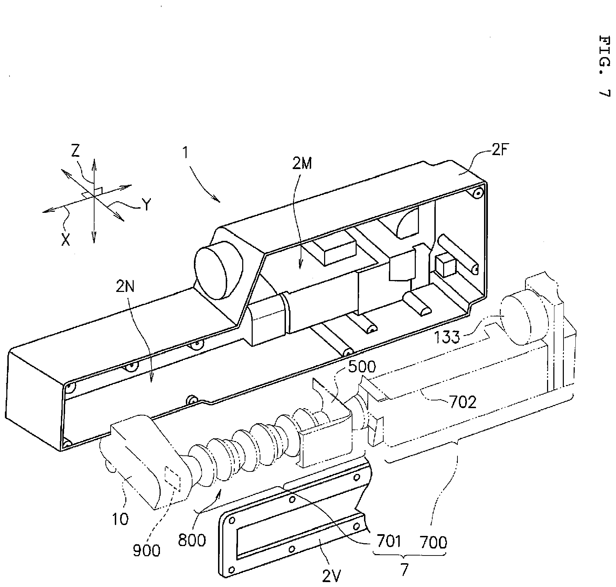

Fig. 7 is an exploded perspective view illustrating the front cover 2F, the syringe plunger drive unit 7, the main body flange pressing portion 500, the syringe plunger pressuring member 10, and a cover member 2V seen from behind. Fig. 7 illustrates the inner surface side of the front cover 2F. The front cover 2F has a main accommodating portion 2M and an extension portion 2N which is formed so as to laterally extend from the main accommodating portion 2M. Inside the main accommodating portion 2M and the extension portion 2N, the syringe plunger drive unit 7, the main body flange pressing portion 500, and the boot 800 are contained. The cover member 2V is fixed to the extension portion 2N by a screw.

The syringe plunger drive unit 7 illustrated in Fig. 7 has a drive unit main body 700, the syringe plunger pressuring member 10, and a pressuring operation portion 701 which is connected to the syringe plunger pressuring member 10. The drive unit main body 700 is contained in a lower portion inside the main accommodating portion 2M of the front cover 2F. The pressuring operation portion 701 and the syringe plunger pressuring member 10 are contained inside the extension portion 2N.

Subsequently, an example of the shape of the boot 800 as a cover member illustrated in Figs. 5 and 6 will be described.

The boot 800 is a member which is elastically deformed so as to be contractible when delivering a drug solution inside the syringe main body 201 by pressing the syringe plunger 202 illustrated in Fig. 2 toward the syringe main body 201. As illustrated in Fig. 5, the boot 800 is placed between the right side surface portion 8V of the syringe main body holding unit 8D of the accommodating portion 8 and a main body portion 80 of the syringe plunger pressuring member 10. The boot 800 is made of rubber or plastic, for example, which is stretchable and contractible due to elastic deformation. The boot can stretch and contract in accordance with a movement of the syringe plunger pressuring member 10 in an X1-direction and an X2-direction.

The boot 800 has a splash-proof structure so as to cover machine elements such as the feed screw 135 or the like illustrated in Fig. 4. Accordingly, for example, even though a drug solution inside the syringe main body 201 overflows, an instillation fluid placed above overflows, or an antiseptic solution, water or the like used in the vicinity thereof is scattered, the machine elements such as the feed screw 135 or the like are prevented against adhering of the solutions or fluid.

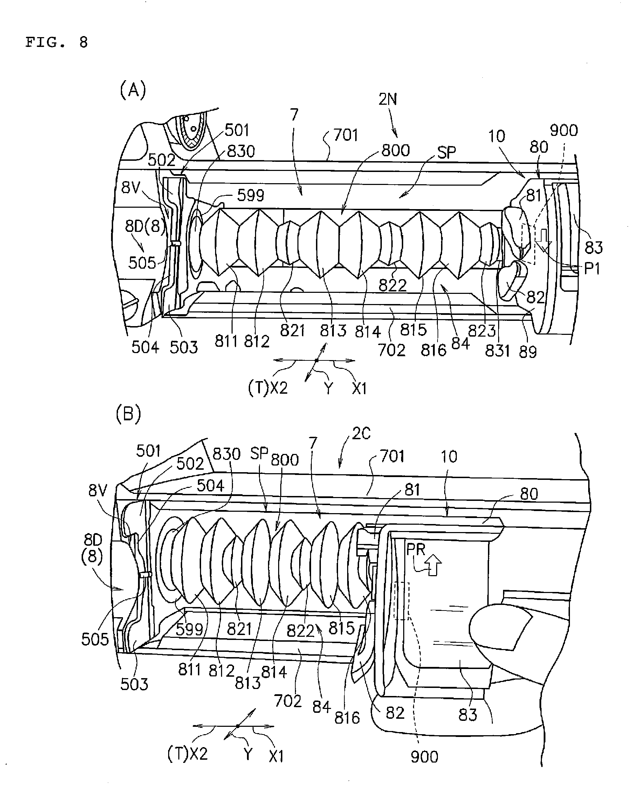

Fig. 8 is a front view including a portion of a cross section illustrating examples of the shape of the boot 800. As exemplified in Fig. 8, the boot 800 has a plurality of first convex portions 811, 812, 813, 814, 815, and 816 having the same sizes, a plurality of second convex portions 821, 822, and 823 having the same sizes, and connection portions 830 and 831 on the right and left, for example. The diameters of the second convex portions 821, 822, and 823 are smaller than the diameters of the first convex portions 811, 812, 813, 814, 815, and 816.

As illustrated in Fig. 6, the connection portion 830 on the left is fixed to the syringe main body holding unit 8D on the right side surface portion 8V side through the hole portion 599 of the main body flange grasping portion 500.

As illustrated in Fig. 8, two adjacent first convex portions 811 and 812 are continuously formed next to the connection portions 830 on the left, and one second convex portion 821 is continuously formed next to the first convex portion 812. Two adjacent first convex portions 813 and 814 are continuously formed next to the one second convex portion 821, and one second convex portion 822 is continuously formed next to the first convex portion 814. Moreover, two adjacent first convex portions 815 and 816 are continuously formed next to the one second convex portion 822, and the one second convex portion 823 is continuously formed next to the first convex portion 816. The one second convex portion 823 is connected to the main body portion 80 on an inner side surface 89 side via the connection portion 831 on the right side.

Accordingly, as the syringe plunger pressurizing member 10 moves to the left and the boot 800 is elastically deformed so as to be contracted, each of the second convex portions 821, 822, and 823 enters the first convex portion having the greater diameter.

Subsequently, a structural example of the syringe plunger drive unit 7 illustrated in Fig. 2 will be described with reference to Fig. 8. Fig. 8 (A) illustrates a state where the boot 800 is stretched to the maximum, and Fig. 8(B) illustrates a state where the plunger flange 205 is nearly fixed to the syringe plunger pressuring member 10.

As illustrated in Figs. 2 and 8, the syringe plunger drive unit 7 is contained inside the extension portion 2N of the main body cover 2, thereby being held therein. The extension portion 2N is formed so as to extend from below the main body cover 2 in the X1-direction. As illustrated in Fig. 2, the extension portion 2N has an upper side surface portion 701, a lower side surface portion 702, and a right side surface portion 703. The extension portion 2N has a space SP surrounded by the upper side surface portion 701, the lower side surface portion 702, the right side surface portion 703, and the right side surface portion 8V of the syringe main body holding unit 8D of the accommodating portion 8 which has been described in Fig. 8. The syringe plunger drive unit 7 is contained inside the space SP.

As illustrated in Fig. 2, the syringe plunger pressuring member 10 of the syringe plunger drive unit 7 gradually presses the plunger flange 205 of the syringe plunger 202 relatively in the T-direction (the X2-direction) against the syringe main body 201, in response to a command from the control unit 100 in Fig. 4. The feed screw 135 is an example of a guide member for guiding the syringe plunger pressuring member 10 in the T-direction (the X2-direction).

As the drive motor 133 of the syringe plunger drive unit 7 illustrated in Fig. 4 is driven in response to a command from the control unit 100, the drive motor 133 rotates the feed screw 135, thereby moving the syringe plunger pressuring member 10 in the T-direction. Accordingly, the syringe plunger pressurizing member 10 presses the syringe plunger 202 in the T-direction, and thus, a drug inside the syringe main body 201 illustrated in Fig. 2 can be properly delivered to the patient P through the tube 203 via the indwelling needle 204.

As illustrated in Fig. 8, the syringe plunger pressuring member 10 includes the plastic main body portion 80, two grasping members 81 and 82, and the operation lever 83. The main body portion 80 is movable along a guide rail 84 of the extension portion 2N in the X1-direction and the X2-direction (the T-direction). The two grasping members 81 and 82 grasp a portion in the vicinity of the plunger flange 205 of the syringe plunger 202 illustrated in Fig. 2, so as to prevent the plunger flange 205 from coming off the syringe plunger pressuring member 10. A health care worker can press the operation lever 83 down with one's finger against biasing force of a spring from the initial position illustrated in Fig. 8 (A) in a P1-direction and can cause the operation lever 83 to be lifted by biasing force of the spring so as to return to the initial position in a PR-direction, as illustrated in Fig. 8(B).

As indicated by the dotted line, the pressure sensor 900 is placed in the main body portion 80 and is positioned between the two grasping members 81 and 82. Accordingly, in a state where the two grasping members 81 and 82 grasp the portion in the vicinity of the plunger flange 205 illustrated in Fig. 2, the pressure sensor 900 directly receives pressure from the plunger flange 205 illustrated in Fig. 2, thereby being able to detect the pressure.

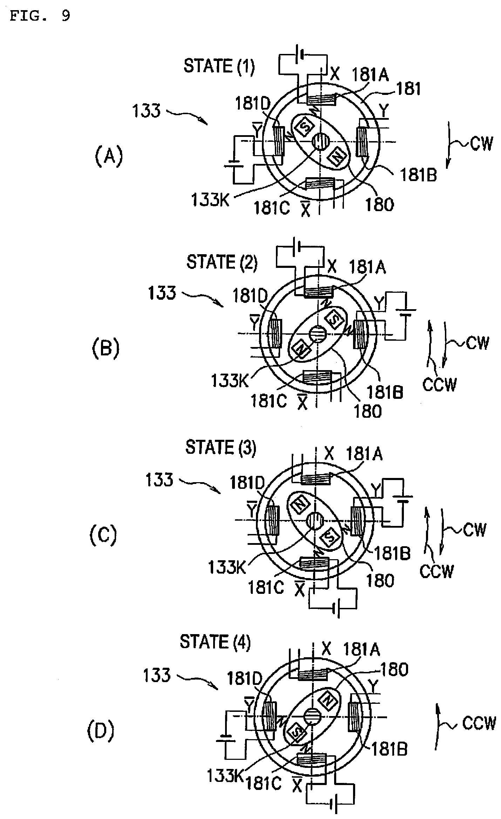

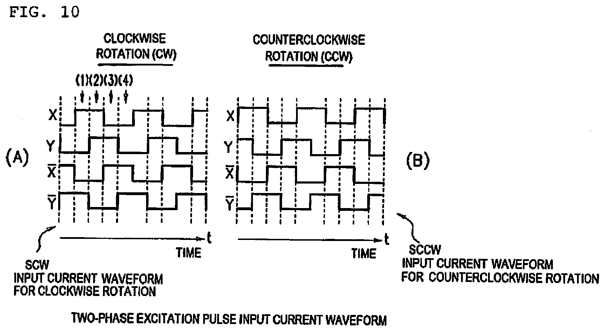

Subsequently, an example of the drive motor 133 illustrated in Fig. 4 will be described with reference to Figs. 9 and 10.

Fig. 9 illustrates a structural example of the drive motor 133, and Fig. 10 illustrates the pulse input current waveforms SCW and SCCW applied to the drive motor 133 illustrated in Fig. 9.

For example, the drive motor 133 illustrated in Fig. 9 is a two-phase quadrupole-type step motor and has a magnet rotor 180 and an electromagnet stator 181. The magnet rotor 180 is a permanent magnet having the N-pole and the S-pole and is rotatable in the stator 181. The magnet rotor 180 is fixed to an output shaft 133K. The stator 181 has four excitation coils, that is, coils 181A, 181B, 181C, and 181D which are placed every 90-degree in the clockwise direction, for example. The coil 181A is an X-terminal, the coil 181B is a Y-terminal, the coil 181C is an X-bar terminal, and the coil 181D is a Y-bar terminal.

When the two-phase excitation pulse input current waveform SCW for clockwise rotation illustrated in Fig. 10(A) is applied from the motor driver 134 to the coils 181A, 181B, 181C, and 181D of the drive motor 133 illustrated in Fig. 9 in response to the command CT from the control unit 100 illustrated in Fig. 4, the magnet rotor 180 rotates in a clockwise rotation direction CW as illustrated in Figs. 9(A) to 9(D) . In other words, when being shifted from a state where the input current waveform SCW is supplied to the coils 181A and 181D as illustrated in Fig. 9(A) to a state where the input current waveform SCW is supplied to the coils 181A and 181B as illustrated in Fig. 9(B), the magnet rotor 180 rotates clockwise in the clockwise rotation direction CW by 90 degrees.

Then, when being shifted from the state where the input current waveform is supplied to the coils 181A and 181B as illustrated in Fig. 9(B) to a state where the input current waveform SCW is supplied to the coils 181B and 181C as illustrated in Fig. 9(C), the magnet rotor 180 rotates further clockwise in the clockwise rotation direction CW by 90 degrees. Moreover, when being shifted from the state where the input current waveform SCW is supplied to the coils 181B and 181C as illustrated in Fig. 9(C) to a state where the input current waveform SCW is supplied to the coils 181C and 181D as illustrated in Fig. 9(D), the magnet rotor 180 rotates further clockwise in the clockwise rotation direction CW by 90 degrees. In this manner, the magnet rotor 180 can continuously rotate in the clockwise rotation direction CW with respect to the stator 181.

When the two-phase excitation pulse input current waveform SCCW for counterclockwise rotation illustrated in Fig. 10(B) is applied from the motor driver 134 to the coils 181A, 181B, 181C, and 181D of the drive motor 133 illustrated in Fig. 9 in response to the command CT from the control unit 100 illustrated in Fig. 4, the magnet rotor 180 rotates in a counterclockwise rotation direction CCW as illustrated in Figs. 9(D) to 9(A).

In this manner, in the drive motor 133, only when the pulse input current waveforms SCW and SCCW are applied from the motor driver 134 in response to the command CT of the control unit 100 illustrated in Fig. 4, the magnet rotor 180 can rotate clockwise or counterclockwise every 90-degree, and thus, the control unit 100 can control the rotation speed and the rotation direction of the magnet rotor 180. As illustrated in Figs. 9 (A) to 9 (D), the magnet rotor 180 is stable at an intermediate position between two poles of the stator 181, and when the magnet rotor 180 rotates, the magnetism is repulsed due to inversion of the stator polarity on the opposite side in the rotation direction, and thus, the magnet rotor 180 can rotate clockwise or counterclockwise. Moreover, since the drive motor 133 is the step motor, the angle in the rotation direction of the magnet rotor 180 can be accurately controlled. In a paused state, since the magnet rotor 180 is fixed due to the magnetism, the pausing force of the magnet rotor 180 is great, and thereby is suitable for stopping at a certain angle.

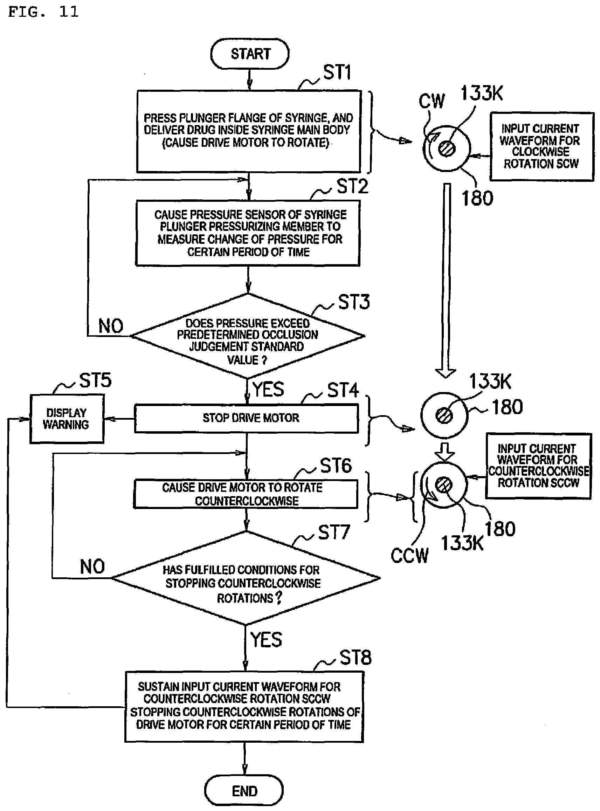

Subsequently, an example of use of the syringe pump 1 according to the embodiment of the present invention will be described with reference to Fig. 11.

Fig. 11 is a flow chart illustrating an operational example of the drive motor 113 of the syringe pump 1.

In Step ST1 illustrated in Fig. 11, a health care worker selects the syringe 200, for example, containing a drug from the syringes 200, 300, and 400 in which multiple types of drugs are respectively contained as illustrated in Fig. 3. Then, as illustrated in Figs. 1 and 2, the syringe 200 is mounted in the syringe pump 1. As illustrated in Fig. 2, a health care worker causes the syringe main body 201 to be contained inside the syringe main body holding unit 8D of the accommodating portion 8, and causes the tube 203 to be fixed by the clamp 5 while being fitted inside the tube fixing portion 9. The syringe main body 201 can be fixed inside the syringe main body holding unit 8D of the accommodating portion 8. Moreover, a portion of the main body flange 209 is grasped by the main body flange pressing portion 500.

As illustrated in Fig. 8(A), a health care worker presses the syringe plunger pressurizing member 10 in the T-direction (the X2-direction) as illustrated in Fig. 8(B) while keeping the operation lever 83 pressed in the P1-direction with one's finger. Accordingly, the main body portion 80 of the syringe plunger pressuring member 10 is caused to approach the plunger flange 205 as illustrated in Fig. 2, and the plunger flange 205 of the syringe plunger 202 is grasped by the two grasping members 81 and 82 of the syringe plunger pressuring member 10 illustrated in Fig. 8.

As the syringe main body 201 is fixed inside the syringe main body holding unit 8D of the accommodating portion 8, a syringe recognition signal CR indicating that the potentiometer 122 illustrated in Fig. 4 has detected the syringe main body 201 of the syringe 200 is transmitted to the control unit 100. Thus, the control unit 100 discriminates that the syringe main body 201 is mounted for example, among the multiple types of syringe main bodies such as the syringe main bodies 201, 301, and 401 illustrated in Fig. 3. Moreover, since the pressure sensor 900 is pressed by the plunger flange 205 of the syringe 200, the pressure measurement signal PS is transmitted from the pressure sensor 900 to the control unit 100.

When the drive motor 133 of the syringe plunger drive unit 7 illustrated in Fig. 4 applies the two-phase excitation pulse input current waveform SCW for clockwise rotation illustrated in Fig. 10 (A) from the motor driver 134 in response to the command CT from the control unit 100, the magnet rotor 180 and the output shaft 133K of the output shaft 113K drive motor 133 in Fig. 9 continuously rotate in the clockwise rotation direction CW as illustrated in Figs. 9(A) to 9(D).

The output shaft 133K of the magnet rotor 180 illustrated in Fig. 9(A) is connected to the feed screw 135 illustrated in Fig. 4 by a gear device (not illustrated). Accordingly, as the feed screw 135 illustrated in Fig. 4 rotates in accordance with rotations of the output shaft 133K, the feed screw 135 can move the syringe plunger pressuring member 10 slightly in the T-direction. Accordingly, the syringe plunger 202 can properly deliver a drug solution inside the syringe main body 201 illustrated in Fig. 2 to the patient P through the tube 203 via the indwelling needle 204.

Subsequently, in Step ST2 illustrated in Fig. 11, while a drug inside the syringe main body 201 is delivered to the patient P as described above, the pressure sensor 900 is pressed by the plunger flange 205 of the syringe 200. Thus, the control unit 100 receives the pressure measurement signal PS from the pressure sensor 900 at a predetermined time interval, for example, every 50 milliseconds.

In Step ST3, the control unit 100 judges whether the transmitted pressure measurement signal PS is equal to or less than a predetermined occlusion judgement standard value LC of the tube 203 or exceeds the occlusion judgement standard value LC. Regarding the occlusion judgement standard value LC, it is assumed that a portion of the tube 203 is bent or twisted resulting in occlusion of the tube 203, for example, depending on a positional relationship between the syringe pump 1 and the patient P, for example, and the value LC is able to be acquired by actually surveying such a state in advance, for example.

As a value for the predetermined occlusion judgement standard value LC in respect to the tube 203, for example, occlusion pressure is 300 mmHg (however, in accordance with a usage environment, the value may be set by being selected from three stages such as "L": 200 mmHg, "MH": 300 mmHg, and "H": 500 mmHg, or the value may be arbitrarily set within a range from 200 mmHg to 300 mmHg). In this case, the control unit 100 adopts a movement average pressure value out of the multiple pressure measurement signals PS, thereby acquiring an occlusion value of the tube 203. The control unit 100 compares the acquired occlusion value and the occlusion pressure judgement standard value LC. The control unit 100 adopts a movement average based on twenty pressure measurement signals PS, for example, so that the average movement value of the pressure measurement signals PS can be referred to as the occlusion value of the tube 203. Accordingly, the occlusion value of the tube 203 can prevent noise from occurring.

In the case where the acquired occlusion pressure exceeds the predetermined occlusion judgement standard value LC, for example, the occlusion pressure exceeds 300 mmHg denotes that a portion of the tube 203 is bent or twisted resulting in occlusion of the tube 203, depending on a positional relationship between the syringe pump 1 and the patient P, for example. Note that, the occlusion pressure judgement standard value LC is a value including a portion of sliding friction at the time of sliding of the syringe plunger pressuring member 10 in the T-direction.

Regarding a value for quantity of a drug which can be aspirated from the inside of the tube 203 as a result of occlusion mitigation treatment performed by rotating the drive motor 133 counterclockwise, the value can be selected by the control unit 100 in accordance with the size of the syringe which is set in the syringe pump 1.

The control unit 100 in Fig. 4 has a table of integrated quantities of aspirating drugs of which the values differ in accordance with the multiple types of syringes 200, 300, and 400 illustrated in Fig. 3 and the manufacturer thereof. The syringe recognition signal CR from the potentiometer 122 allows the control unit 100 to discriminate which volume of the syringe among the multiple types of syringe main bodies 201, 301, and 401 illustrated in Fig. 3 is mounted, for example, and the manufacturer thereof can be selected through the menu selection button 4E. Thus, in this example, the control unit 100 can select the integrated quantity of a aspirating drug corresponding to the discriminated syringe main body 201 having the largest volume, for example, from the table of integrated quantities of drugs. Note that, the name of manufacturer may be stored in RFID provided in each of the plunger flanges 205, 305, and 405 so as to be automatically read by a reader.

In Step ST3, if the occlusion value of the pressure measurement signal PS is equal to or less than the predetermined occlusion judgement standard value LC of the tube 203 in Step ST3, the control unit 100 returns to Step ST2. If the pressure measurement signal PS exceeds the predetermined occlusion judgement standard value LC of the tube 203 in Step ST3, the control unit 100 judges that a portion of the tube 203 in Fig. 2 is bent or twisted depending on the positional relationship between the syringe pump 1 and the patient P and the portion of the tube 203 is in an occlusion state, thereby proceeding to Step ST4.

Therefore, in Step ST4, in response to the command CT of the control unit 100, the motor driver 134 stops applying the two-phase excitation pulse input current waveform SCW for clockwise rotation illustrated in Fig. 10(A), and thus, the magnet rotor 180 of the drive motor 133 stops rotating clockwise.

When the magnet rotor 180 of the drive motor 133 stops rotating clockwise in Step ST4, as necessary at this time, in Step ST5, the control unit 100 in Fig. 4 issues a command to the display unit driver 130 so as to cause the display unit 3 to display a warning that "the drive motor has stopped", and a display screen of the display unit 3 varies from "a yellow background" to "a white background", for example, thereby alerting a health care worker. The control unit 100 issues acoustic notification regarding "the stopping of the drive motor" through the speaker 131.

In Step ST5, since a portion of the tube 203 is bent or twisted depending on the positional relationship between the syringe pump 1 and the patient P so that the tube 203 is occluded, the control unit 100 in Fig. 4 instantly rotates the magnet rotor 180 of the drive motor 133 in Fig. 9 in the counterclockwise rotation direction CCW. The reason for rotating the drive motor 133 counterclockwise is as follows. That is, because there is a concern that when a health care worker is aware of a portion of the tube 203 which is bent or twisted, through the warning display on the display unit 3 or the acoustic notification, and the health care worker straightens the bent or twisted portion of the tube 203 so as to relieve the tube 203 from the occlusion state, the occlusion inside the tube 203 may be immediately released so that a drug inside the tube 203 may be abruptly sent to the patient P.

Therefore, in order to prevent the drug from being abruptly sent to the patient P, the control unit 100 in Fig. 4 causes the magnet rotor 180 of the drive motor 133 in Fig. 9 to rotate once in the counterclockwise rotation direction CCW. When rotating the magnet rotor 180 counterclockwise, the magnet rotor 180 rotates counterclockwise at a rotational frequency of 2,000 pps, for example, thereby lowering the pressure inside the tube 203. Accordingly, even though a health care worker restores the bent or twisted state of the portion of the tube 203 so as to relieve an occlusion state of the tube 203, a drug is prohibited from being instantly sent to the patient P. Therefore, it is possible to establish countermeasures for rapid bolus injection (injection of a drug in a short period of time) when cancelling an occlusion state of the tube 203.

In Step ST7 of Fig. 11, the judgement unit 100 judges whether or not predetermined conditions for stopping the counterclockwise rotation in order to perform occlusion mitigation treatment are fulfilled. A plurality of the predetermined conditions for stopping the counterclockwise rotation as follows: (1) occlusion pressure is equal to or less than a predetermined value (50 mmHg), (2) "quantity of counterclockwise rotations" determined for each syringe reaches a predetermined number of steps, (3) integrated quantity is equal to or less than integrated quantity at the time of starting delivering a drug, (4) integrated quantity is zero, or the like.

Here, the integrated quantity denotes the entire quantity of a drug delivered to a patient after very new syringes 200, 300, and 400 containing predetermined drugs are placed in a syringe placement portion of the syringe pump 1 so as to start delivering the drug. Depending on the size of the mounted syringe, the quantity of counterclockwise rotations of the magnet rotor 180 of the drive motor 133 in Fig. 9 rotating in the counterclockwise rotation direction CCW varies. That is, as described above, for example, since the control unit 100 recognizes mounting status of the syringe main body 201, in accordance with the size of the syringe main body 201 illustrated in Fig. 2, for example, the control unit 100 in Fig. 4 instructs the motor driver 134 regarding the quantity of counterclockwise rotations of the magnet rotor 180 of the drive motor 133 in the counterclockwise rotation direction CCW, thereby decreasing pressure inside the tube 203 in accordance with the size of the syringe main body 201. However, when decreasing pressure in the tube 203, only the pressure force which is generated due to occlusion of the tube 203 is to be relieved, and the inside of the tube 203 needs to be prevented from being under negative pressure. In other words, when rotating the magnet rotor 180 counterclockwise, the magnet rotor 180 is caused to rotate counterclockwise as many times as the number of steps in "quantity of counterclockwise rotations" which is determined for each predetermined syringe.

If the inside of the tube 203 is under negative pressure, there is a concern that for example, blood or the like of the patient P may be aspirated to the syringe main body 201 through the tube 203. Therefore, in order to adjust the pressure inside the tube 203 so as not to cause the inside of the tube 203 to be under negative pressure, the control unit 100 needs to set the quantity of counterclockwise rotations of the magnet rotor 180 in the counterclockwise rotation direction CCW.

In Step ST8 of Fig. 11, if any one of the predetermined conditions for stopping the counterclockwise rotation in accordance with the size of the syringe main body 201 of the syringe 200 is fulfilled, the magnet rotor 180 of the drive motor 133 stops rotating counterclockwise. At the time of stopping, the motor driver 134 in Fig. 4 keeps applying the input current waveform SCCW for counterclockwise rotation to the drive motor 133 for a certain period of time. That is, when stopping a counterclockwise rotation operation of the drive motor 133 by issuing the command CT to the motor driver 134, the control unit 100 causes the drive motor 133 to maintain excitation of the counterclockwise rotation operation for a certain period of time, and then, the counterclockwise rotation operation of the drive motor 133 is stopped.

For example, if the state (2) in Fig. 9(B) is a state where the counterclockwise rotation operation is stopped, the control unit 100 issues the command CT to the motor driver 134 so as to maintain the application state (the excitation state) of the input current waveform in the state (2) for only a certain period of time. Thereafter, the input current waveform SCCW is stopped from being applied. The certain period of time denotes a period to the extent that for example, the magnet rotor 180 does not rotate further in the counterclockwise rotation direction CCW due to inertia, and preferably a period ranging approximately from 10 ms to 100 ms. Accordingly, the magnet rotor 180 is prevented from rotating in the counterclockwise rotation direction CCW due to inertia, and the occlusion pressure inside the tube 203 is prevented from being lowered further.

In this manner, when the drive motor 133 stops the counterclockwise rotation operation in Step ST7, as illustrated in Step ST5, the control unit 100 in Fig. 4 issues a command to the display unit driver 130 so as to cause the display unit 3 to display a warning that "the drive motor has stopped", and the display screen of the display unit 3 varies from "a yellow background" to "a white background", thereby alerting a health care worker. The control unit 100 issues acoustic notification regarding "the stopping of the drive motor" through the speaker 131 or the lamp 4A which is being lit or blinking red.

Due to a certain reason, when the photo-coupler sensor 250 in Fig. 4 detects that the main body flange 209 of the syringe 200 illustrated in Fig. 2 has come off from a state of being grasped by the main body flange pressing portion 500, the control unit 100 receives a main body flange coming-off signal GK from the photo-coupler sensor 250. In this case, the control unit 100 causes the magnet rotor 180 of the drive motor 133 to stop rotating counterclockwise and issues a warning by using the speaker 131, the lamp 4A which is being lit or blinking red, or the display unit 3, which is a notification unit. Accordingly, in spite of the syringe 200 being not mounted in the syringe pump in a complete state, it is possible to discontinue pressure inside the tube 203 from being lowered.

Moreover, due to a certain reason, when the plunger flange 205 has come off from the two grasping members 81 and 82 of the syringe plunger pressurizing member 10 illustrated in Fig. 8, the plunger flange 205 is no longer attached to the pressure sensor 900, and the pressure measurement signal PS of the pressure sensor 900 disappears. In this case as well, the control unit 100 causes the magnet rotor 180 of the drive motor 133 to stop rotating counterclockwise and issues a warning by using the speaker 131, the lamp 4A which is being lit or blinking red, or the display unit 3, which is the notification unit.

In these cases as well, as illustrated in Step ST5, the control unit 100 in Fig. 4 issues a command to the display unit driver 130 so as to cause the display unit 3 to display a warning that "the drive motor has stopped", and the display screen of the display unit 3 varies from "a yellow background" to "a white background", thereby alerting a health care worker. The control unit 100 issues acoustic notification regarding "the stopping of the drive motor" through the speaker 131 or the lamp 4A which is being lit or blinking red.

According to the embodiment of the present invention, the syringe pump 1 delivers a drug inside the syringe to a patient via the tube by pressing the syringe plunger of the syringe filled with the drug. The syringe pump includes the syringe setting portion in which the syringe main body of the syringe is set; the drive motor; the movement member that presses the syringe plunger in accordance with a clockwise rotation operation of the drive motor so as to deliver the drug inside the syringe to the patient; the pressure sensor that detects pressure while pressing the syringe plunger, in order to detect that the tube is in an occluded state; and the control unit that causes the drive motor to perform a counterclockwise rotation operation when pressure detected by the pressure sensor exceeds the predetermined occlusion judgement standard value, and causes the drive motor to stop the counterclockwise rotation operation when pressure detected by the pressure sensor becomes equal to or less than the predetermined occlusion judgement standard value.

Accordingly, the pressure sensor detects pressure at the time of pressing the syringe plunger, in order to detect that the tube is in an occluded state. The control unit causes the drive motor to perform a counterclockwise rotation operation when pressure detected by the pressure sensor exceeds the predetermined occlusion judgement standard value, and the control unit causes the drive motor to stop the counterclockwise rotation operation when pressure detected by the pressure sensor becomes equal to or less than the predetermined occlusion judgement standard value.

In other words, when the pressure detected by the pressure sensor exceeds the predetermined occlusion judgement standard value, it is considered that there is an occurrence of occlusion caused by bending or twisting of the tube. Therefore, there is a possibility that large quantity of a drug solution stagnates at a certain place if the drug solution is continuously delivered. In this case, the drive motor is caused to rotate counterclockwise, thereby preventing such a situation. When the pressure detected by the pressure sensor becomes equal to or less than the predetermined occlusion judgement standard value, it is assumed that bending or twisting of the tube has been relieved. Therefore, even though there is a drug solution stagnating at a certain place in the tube, it is possible to relieve the stagnation state thereof.

Accordingly, when an occlusion state of the tube is cancelled by straightening bending or twisting of the tube, delivery of a drug can be discontinued while preventing the drug from abruptly flowing inside the tube, and thus, the safety of an operation of delivering the drug can be enhanced.

The drive motor is the step motor, and the pressure sensor is placed in the movement member. Therefore, by adopting the step motor as the drive motor, the drive motor can be easily shifted from a clockwise rotation operation to a counterclockwise rotation operation. Moreover, the pressure sensor can directly receive pressure from the syringe plunger. Thus, it is possible to easily detect the occlusion state of the tube.

The control unit causes excitation of the drive motor to be maintained for a certain period of time when stopping the counterclockwise rotation operation of the drive motor. Therefore, the rotor can be prevented from rotating any further counterclockwise due to inertia of the rotor when stopping the counterclockwise rotation operation of the rotor of the drive motor. Thus, a drug inside the tube is unlikely to be subjected to unnecessary aspirating.

There is provided the notification unit which issues notification regarding stopping of the drive motor if the drive motor stops. Therefore, the notification unit issues notification regarding the stopping of the drive motor. Thus, it is possible for a health care worker to confirm discontinuance of the operation of delivering a drug caused by the stopping of the drive motor.

The notification unit is the speaker which performs an acoustic announcement regarding stopping of the drive motor. Therefore, the speaker issues acoustic notification regarding the stopping of the drive motor. Thus, it is possible for a health care worker to acoustically confirm discontinuance of the operation of delivering a drug caused by the stopping of the drive motor.

The notification unit is the display unit which performs a display regarding stopping of the drive motor. Therefore, the display unit issues notification regarding the stopping of the drive motor. Thus, it is possible for a health care worker to visually confirm discontinuance of the operation of delivering a drug caused by the stopping of the drive motor.

The display unit displaying information and the operation panel section having the operation button are placed at an upper portion of the main body of the syringe pump, and the syringe setting portion and the movement member are placed at a lower portion of the main body of the syringe pump. Accordingly, a health care worker can perform the task of delivering a drug solution from the syringe while confirming information on the display unit at the upper portion of the main body. Thus, it is possible for a health care worker to operate the operation button of the operation panel section while confirming the information on the display unit at the upper portion of the main body.

The present invention is not limited to the above-described embodiment, and various changes can be made thereto without departing from the scope of Claims. In each configuration in the above-described embodiment, a portion thereof can be omitted and can be arbitrarily combined so as to be unique.

The drive motor 133 may be a three-phase step motor, for example, and not the two-phase step motor.

### Reference Signs List

1 ... SYRINGE PUMP,
2 ... CASING,
6 ... SYRINGE PLACEMENT PORTION,
7 ... SYRINGE PLUNGER DRIVE UNIT,
10 ... SYRINGE PLUNGER PRESSURIZING MEMBER (MOVEMENT MEMBER),
80 ... MAIN BODY PORTION,
133 ... DRIVE MOTOR,
180 ... MAGNET ROTOR,
181 ... STATOR,
200, 300, 400 ... SYRINGE,
201, 301, 401 ... SYRINGE MAIN BODY,
202, 302, 402 ... SYRINGE PLUNGER,
205, 305, 405 ... PLUNGER FLANGE,
209, 309, 409 ... MAIN BODY FLANGE, AND
900 ... PRESSURE SENSOR.

## Claims

1. A syringe pump (1) which is configured to deliver a drug inside a syringe (200, 300, 400) to a patient via a tube (203) by pressing a syringe plunger (202, 302, 402) of the syringe (200, 300, 400) filled with the drug, the pump (1) comprising:
a syringe setting portion (6) in which a syringe main body (201, 301, 401) of the syringe (200, 300, 400) can be placed;
a drive motor (133) which is a step motor including a magnet rotor (180);
a movement member (10) that is configured to press the syringe plunger (202, 302, 402) in accordance with a clockwise rotation operation of the magnet rotor (180) of the drive motor (133) so as to deliver the drug inside the syringe (200, 300, 400) to a patient;
a pressure sensor (900) that is configured to detect pressure while pressing the syringe plunger (202, 302, 402), in order to detect that the tube (203) is in an occluded state;
a potentiometer (122) that is configured to discriminate the sizes of the syringe (200, 300, 400); and
a control unit (100) that is configured to perform occlusion mitigation treatment by starting counterclockwise rotation of the magnet rotor (180) of the drive motor (133) when pressure detected by the pressure sensor (900) exceeds a predetermined occlusion judgement standard value (LC), and subsequently stop a counterclockwise rotation operation of the magnet rotor (180) of the drive motor (133) if any one of four predetermined conditions for stopping the counterclockwise rotation of the magnet rotor (180) is fulfilled,
**characterized in that**
the syringe (200, 300, 400) of a necessary capacity can be mounted in the syringe pump (2), and
the control unit (100) is configured to cause excitation of the counterclockwise rotation operation of the magnet rotor (180) of the drive motor (133) to be maintained for a certain period of time before stopping the counterclockwise rotation operation of the magnet rotor (180) of the drive motor (133),
wherein the four predetermined conditions for stopping the counterclockwise rotation of the magnet rotor (180) of the drive motor (133) by the control unit (100) which the control unit (100) is configured to verify are the following (1) to (4):
(1) Occlusion pressure for the placed syringe (200, 300, 400) transmitted from the pressure sensor (900) to the control unit (100) and judged by the control unit (100) is equal to or less than a predetermined value of 50 mmHg stored in the control unit (100),
(2) "Quantity of counterclockwise rotations" of the magnet rotor (180) determined by the control unit (100) for the placed syringe (200, 300, 400) reaches a predetermined number of steps determined by the control unit (100) depending on the size of the placed syringe (200, 300, 400) discriminated by the potentiometer (122),
(3) Integrated quantity measured by the control unit (100) is equal to or less than integrated quantity measured by the control unit (100) at the time of starting delivering a drug, and
(4) Integrated quantity measured by the control unit (100) is zero.

2. The syringe pump (1) according to Claim 1,
wherein the pressure sensor (900) is placed in the movement member (10).

3. The syringe pump (1) according to Claim 2,
wherein the control unit (100) is configured to cause excitation of the counterclockwise rotation operation of the drive motor (133) to be maintained for a certain period of time when stopping the counterclockwise rotation operation of the drive motor (133).

4. The syringe pump (1) according to Claim 1, further comprising:
a notification unit that issues notification regarding stopping of the drive motor (133) if the drive motor (133) stops.

5. The syringe pump (1) according to Claim 4,
wherein the notification unit is a speaker (131) which performs an acoustic announcement regarding stopping of the drive motor (133).

6. The syringe pump (1) according to Claim 4,
wherein the notification unit is a display unit (3) which performs a display regarding stopping of the drive motor (133).

7. The syringe pump (1) according to Claim 6,
wherein the display unit (3) displaying information and an operation panel section (4) having an operation button are configured to be placed at an upper portion of a main body (201, 301, 401) of the syringe pump (1), and the syringe setting portion (6) and the movement member (10) are configured to be placed at a lower portion of the main body (201, 301, 401) of the syringe pump (1).

## Patentansprüche

1. Spritzenpumpe (1), die so konfiguriert ist, dass sie ein Arzneimittel innerhalb einer Spritze (200, 300, 400) durch Drücken eines Spritzenkolbens (202, 302, 402) von der mit dem Arzneimittel gefüllten Spritze (200, 300, 400) über einen Schlauch (203) an einen Patienten abgibt, wobei die Pumpe (1) umfasst:
einen Spritzeneinsetzabschnitt (6), in den ein Spritzenhauptkörper (201, 301, 401) der Spritze (200, 300, 400) eingesetzt werden kann;
einen Antriebsmotor (133), der ein Schrittmotor ist, welcher einen Magnetrotor (180) umfasst;
ein Bewegungselement (10), das so konfiguriert ist, dass es entsprechend einem Drehvorgang von dem Magnetrotor (180) des Antriebsmotors (133) im Uhrzeigersinn den Spritzenkolben (202, 302, 402) drückt, um das Arzneimittel innerhalb der Spritze (200, 300, 400) an einen Patienten abzugeben;
einen Drucksensor (900), der konfiguriert ist, um Druck zu erfassen, während der Spritzenkolben (202, 302, 402) gedrückt wird, um zu erfassen, dass sich der Schlauch (203) in einem verschlossenen Zustand befindet;
ein Potentiometer (122), das so konfiguriert ist, dass es die Größen der Spritze (200, 300, 400) unterscheidet; und
eine Steuereinheit (100), die so konfiguriert ist, dass sie eine Okklusionsminderungsbehandlung durch Starten einer Drehung des Magnetrotors (180) des Antriebsmotors (133) gegen den Uhrzeigersinn durchführt, wenn der durch den Drucksensor (900) erfasste Druck einen vorbestimmten Okklusionsbeurteilungs-Standardwert (LC) überschreitet, und anschließend einen Drehvorgang des Magnetrotors (180) des Antriebsmotors (133) gegen den Uhrzeigersinn stoppt, wenn irgendeine von vier vorbestimmten Bedingungen zum Stoppen der Drehung des Magnetrotors (180) gegen den Uhrzeigersinn erfüllt ist,
**dadurch gekennzeichnet, dass**
die Spritze (200, 300, 400) mit einer erforderlichen Kapazität in der Spritzenpumpe (2) angebracht werden kann, und
die Steuereinheit (100) so konfiguriert ist, dass die Ansteuerung des Drehvorgangs von dem Magnetrotor (180) des Antriebsmotors (133)gegen den Uhrzeigersinn für eine bestimmte Zeitspanne aufrecht gehalten wird, bevor der Linksdrehbetrieb von dem Magnetrotor (180) des Antriebsmotors (133) gestoppt wird,
wobei die vier vorbestimmten Bedingungen zum Stoppen der Drehung von dem Magnetrotor (180) des Antriebsmotors (133) gegen den Uhrzeigersinn durch die Steuereinheit (100), wobei die Steuereinheit (100) konfiguriert ist, diese zu überprüfen, die folgenden (1) bis (4) sind:
(1) der Okklusionsdruck für die eingesetzte Spritze (200, 300, 400), der von dem Drucksensor (900) zur Steuereinheit (100) übertragen und durch die Steuereinheit (100) beurteilt wird, ist gleich oder kleiner als ein vorbestimmter Wert von 50 mmHg, der in der Steuereinheit (100) gespeichert ist,
(2) die durch die Steuereinheit (100) für die eingesetzte Spritze (200, 300, 400) ermittelte "Anzahl von Umdrehungen gegen den Uhrzeigersinn" von dem Magnetrotor (180) erreicht eine durch die Steuereinheit (100) ermittelte vorbestimmte Anzahl von Schritten in Abhängigkeit von der Größe der eingesetzten Spritze (200, 300, 400), die durch das Potentiometer (122) unterschieden wird,
(3) die durch die Steuereinheit (100) gemessene integrierte Anzahl ist gleich oder kleiner als die durch die Steuereinheit (100) bei Beginn der Abgabe eines Arzneimittels gemessene integrierte Anzahl und
(4) die durch die Steuereinheit (100) gemessene integrierte Anzahl ist Null.

2. Spritzenpumpe (1) nach Anspruch 1,
wobei der Drucksensor (900) in dem Bewegungselement (10) angeordnet ist.

3. Spritzenpumpe (1) nach Anspruch 2,
wobei die Steuereinheit (100) konfiguriert ist, um zu bewirken, dass die Ansteuerung des Drehvorgangs des Antriebsmotors (133) gegen den Uhrzeigersinn für eine bestimmte Zeitspanne aufrechtgehalten wird, wenn der Drehvorgang des Antriebsmotors (133) gegen den Uhrzeigersinn gestoppt wird.

4. Spritzenpumpe (1) nach Anspruch 1, ferner umfassend:
eine Benachrichtigungseinheit, die eine Benachrichtigung über das Anhalten des Antriebsmotors (133) ausgibt, wenn der Antriebsmotor (133) anhält.

5. Spritzenpumpe (1) nach Anspruch 4,
wobei die Benachrichtigungseinheit ein Lautsprecher (131) ist, der eine akustische Ansage bezüglich des Anhaltens des Antriebsmotors (133) durchführt.

6. Spritzenpumpe (1) nach Anspruch 4,
wobei die Benachrichtigungseinheit eine Anzeigeeinheit (3) ist, die eine Anzeige bezüglich des Anhaltens des Antriebsmotors (133) durchführt.

7. Spritzenpumpe (1) nach Anspruch 6,
wobei die Anzeigeeinheit (3), die Informationen anzeigt, und ein Bedienfeldabschnitt (4), der eine Bedientaste aufweist, konfiguriert sind, um an einem oberen Abschnitt eines Hauptkörpers (201, 301, 401) der Spritzenpumpe (1) angeordnet zu werden, und der Spritzeneinsetzabschnitt (6) und das Bewegungselement (10) konfiguriert sind, um an einem unteren Abschnitt des Hauptkörpers (201, 301, 401) der Spritzenpumpe (1) angeordnet zu werden.

## Revendications

1. Pompe de seringue (1) qui est conçue pour administrer un médicament contenu à l'intérieur d'une seringue (200, 300, 400) à un patient par l'intermédiaire d'un tube (203) en pressant un piston de seringue (202, 302, 402) de la seringue (200, 300, 400) remplie du médicament, la pompe (1) comprenant :
une partie de fixation de seringue (6) dans laquelle un corps principal de seringue (201, 301, 401) de la seringue (200, 300, 400) peut être placé ;
un moteur d'entraînement (133) qui est un moteur pas à pas comprenant un rotor magnétique (180) ;
un élément mobile (10) qui est conçu pour presser le piston de seringue (202, 302, 402) en fonction d'un mouvement de rotation dans le sens des aiguilles d'une montre du rotor magnétique (180) du moteur d'entraînement (133) de manière à administrer le médicament contenu à l'intérieur de la seringue (200, 300, 400) à un patient ;
un capteur de pression (900) qui est conçu pour détecter la pression tout en pressant le piston de seringue (202, 302, 402), afin de détecter que le tube (203) est dans un état occlus ;
un potentiomètre (122) qui est conçu pour distinguer les tailles de la seringue (200, 300, 400) ; et
une unité de commande (100) qui est conçue pour effectuer un traitement de limitation d'occlusion en démarrant une rotation dans le sens contraire des aiguilles d'une montre du rotor magnétique (180) du moteur d'entraînement (133) lorsque la pression détectée par le capteur de pression (900) dépasse une valeur standard de jugement d'occlusion prédéterminée (LC), et arrêter ensuite une opération de rotation dans le sens contraire des aiguilles d'une montre du rotor magnétique (180) du moteur d'entraînement (133) si l'une quelconque parmi quatre conditions prédéterminées pour arrêter la rotation dans le sens contraire des aiguilles d'une montre du rotor magnétique (180) est satisfaite,
**caractérisée en ce que**
la seringue (200, 300, 400) ayant une capacité nécessaire peut être montée dans la pompe de seringue (2), et
l'unité de commande (100) est conçue pour permettre que l'activation de la rotation dans le sens contraire des aiguilles d'une montre du rotor magnétique (180) du moteur d'entraînement (133) soit maintenue pendant une certaine période de temps avant l'arrêt de l'opération de rotation dans le sens contraire des aiguilles d'une montre du rotor magnétique (180) du moteur d'entraînement (133),
où les quatre conditions prédéterminées pour arrêter la rotation dans le sens contraire des aiguilles d'une montre du rotor magnétique (180) du moteur d'entraînement (133) par l'unité de commande (100), que l'unité de commande (100) est adaptée à vérifier, sont les suivantes (1) à (4) :
(1) la pression d'occlusion de la seringue mise en place (200, 300, 400) transmise depuis la capteur de pression (900) à l'unité de commande (100) et jugée par l'unité de commande (100) est inférieure ou égale à une valeur prédéterminée de 50 mmHg enregistrée dans l'unité de commande (100),
(2) la « quantité de rotations dans le sens contraire des aiguilles d'une montre » du rotor magnétique (180) déterminée par l'unité de commande (100) pour la seringue mise en place (200, 300, 400) atteint un nombre prédéterminé de pas déterminé par l'unité de commande (100) en fonction de la taille de la seringue mise en place (200, 300, 400) discriminée par le potentiomètre (122),
(3) la quantité intégrée mesurée par l'unité de commande (100) est inférieure ou égale à la quantité intégrée mesurée par l'unité de commande (100) au moment de démarrer la distribution d'un médicament, et
4) la quantité intégrée mesurée par l'unité de commande (100) est zéro.

2. Pompe de seringue (1) selon la revendication 1,
dans laquelle le capteur de pression (900) est placé dans l'élément mobile (10).

3. Pompe de seringue (1) selon la revendication 2,
dans laquelle l'unité de commande (100) est conçue pour permettre que l'activation de la rotation dans le sens contraire des aiguilles d'une montre du moteur d'entraînement (133) soit maintenue pendant une certaine période de temps avant l'arrêt de l'opération de rotation dans le sens contraire des aiguilles d'une montre du moteur d'entraînement (133).

4. Pompe de seringue (1) selon la revendication 1, comprenant en outre :
une unité de notification qui émet une notification concernant l'arrêt du moteur d'entraînement (133) si le moteur d'entraînement (133) s'arrête.

5. Pompe de seringue (1) selon la revendication 4,
dans laquelle l'unité de notification est un haut-parleur (131) qui effectue une annonce acoustique concernant l'arrêt du moteur d'entraînement (133).

6. Pompe de seringue (1) selon la revendication 4,
dans laquelle l'unité de notification est une unité d'affichage (3) qui effectue un affichage concernant l'arrêt du moteur d'entraînement (133).

7. Pompe de seringue (1) selon la revendication 6,
dans laquelle l'unité d'affichage (3) affichant des informations et une section de panneau de commande (4) comportant un bouton de commande sont adaptées pour être disposées au niveau d'une partie supérieure d'un corps principal (201, 301, 401) de la pompe de seringue (1), et la partie de fixation de seringue (6) et l'élément mobile (10) sont adaptés pour être disposés au niveau d'une partie inférieure du corps principal (201, 301, 401) de la pompe de seringue (1).
